# EUROPEAN PATENT SPECIFICATION

(11) **EP 2 621 467 B1**
(45) Date of publication and mention of the grant of the patent: **30.08.2017**
(21) Application number: 11820802.4
(22) Date of filing: 22.09.2011
(51) Int. Cl.: A61K 9/00, A61K 41/00, A61L 15/00, A61L 26/00, A61F 13/00, D01F 1/00, D06M 10/00, A41B 9/00, A61K 49/00

(54) **FIBRES AND RELATIVE WOVEN AND NON WOVEN TISSUES FOR THE TOPIC TREATMENT OF SEXUAL DYSFUNCTIONS OF THE MALE GENITAL APPARATUS**
FASERN SOWIE GEWEBTER UND NICHT GEWEBTER STOFF DAMIT ZUR TOPISCHEN BEHANDLUNG VON SEXUELLEN FUNKTIONSSTÖRUNGEN DER MÄNNLICHEN GESCHLECHTSORGANE
FIBRES ET TISSUS TISSÉS ET NON TISSÉS APPARENTÉS POUR LE TRAITEMENT TOPIQUE DE TROUBLES SEXUELS DE L'APPAREIL GÉNITAL MASCULIN

(30) Priority: 27.09.2010 IT MI20101749
(43) Date of publication of application: 07.08.2013
(73) Proprietor: Landi, Federica, 27052 Godiasco Salice Terme (PV) (IT)
(72) Inventor: LANDI, Federica, I-27052 Pavia (IT)
(74) Representative: Asensio, Raffaella Consuelo
(86) International application number: PCT/IB2011/054171
(87) International publication number: WO 2012/042446

(56) References cited:
- EP-A1- 1 219 405
- EP-A1- 1 291 405
- WO-A2-2010/058176
- US-A1- 2009 112 295
- PRIYA PADMANABHAN AND ANDREW R. MCCULLOUGH: "Penile Oxygen Saturation in the Flaccid and Erect Penis in Men With and Without Erectile Dysfunction", JOURNAL OF ANDROLOGY, vol. 28, no. 2, 2007, pages 223-228, XP002635066,

## Description

### FIELD OF THE INVENTION

The present invention concerns fibres containing a powdered composition that is capable of emitting radiation in the far infrared, such fibres, once processed in yarn and woven so as to prepare items of male underwear, or processed in the form of a non-woven fabric, are used for the topical treatment of sexual dysfunctions of the male genital apparatus.

### STATE OF THE ART

Nosography of sexual dysfunctions has recently had considerable and important developments based upon studies of pathology and physiopathology of the male sexual response. Currently, the sexual response cycle is divided into 4 steps: sexual desire, arousal, orgasm and resolution. The Diagnostic and Statistical Manual (DSM-IV) classifies the male sexual disorders into: disorder of the sexual desire, such as hypoactive sexual desire and sexual aversion, the disorders of the sexual arousal such as erectile dysfunction, but also Induratio Penis Plastica (IPP), disorders of the orgasm and resolution, such as premature ejaculation, delayed ejaculation, anejaculation, anorgasmia.

Erectile deficit or dysfunction (ED) defines the incapability of reaching and maintaining a suitable erection so as to be able to have a satisfactory sexual relationship.

It has been estimated that in the USA around 20 million men suffer from such a pathology and analogous data was also found in the European adult male population. In Italy around 3 million males over 18 years old suffer from different degrees of erectile dysfunction.

Up to ten years ago it was suspected that this disorder was mainly caused by psychological factors, whereas, currently and, thanks to the new discoveries in physiology of the erection, it has been possible to find that ED, in most cases, has organic causes, even though it is still problematic to find the actual individual aetiopathogenesis.

Indeed the erection is the result of a delicate combination between psycho-emotional and organic functional aspects and it is sufficient for one or both of them to have problems for ED to occur.

In any case ED, according to the aetiology diagnosed can be classified into the following different categories.

Erectile dysfunction of the vascular type, which can be caused by an atherosclerosis of the penile arteries, or by an inadequate impedance of venous outflow (venous leakage) or to both the causes. Systemic pathologies such as, for example, atherosclerosis, hypertension and diabetes can worsen or possibly lead to ED of the vascular type.

Erectile dysfunctions of the hormonal type are those which are caused by systemic hormonal dysfunctions such as: high prolactin, hypo/hyperthyroidism and also hypogonadism (low testosterone and relatively low bioavailability), even if the aforementioned pathology leads to a decrease in libido (desire) and the relationship between testosterone and erectile capability is not clear.

Erectile dysfunctions from drugs are those caused by the prescription of particular types of drugs and are responsible for around 25% of the cases.

Erectile dysfunctions of neurological origin are those caused by neurological disorders (for example a cerebrovascular accident, epilepsy of the parietal lobe and of the autonomic nervous system, lesions of the spinal cord).

Finally, a further cause of ED is that caused by transurethral resection of the prostate which occurs in 40% of patients undergoing such an operation.

In the cases in which the cause of ED is a disorder of the hormonal type, it can be cured with a suitable hormonal therapy. In any case the hormone prescription can lead to a whole series of contraindications, not least the onset of tumours.

In the other cases the most frequent cures foresee a penile therapy consisting of injecting PGE1, which can lead to a haematoma of the penis and even bleeding and, in any case, must be dosed by the doctor in a suitable manner, so as to avoid the opposite effect or syndrome such as priapism.

The oral treatment with sildenafil, although being effective since it is a drug which works only in a contributory sexual context, is however strongly contraindicated in cardiopathic people treated with nitrates, and moreover causes cephalea in 16% of cases.

The surgical therapy for the arrangement of a penile prosthesis may re-establish the erection, but it can lead to the onset of infections and malfunctioning of the prosthesis.

Induratio Penis Plastica (IPP), better known as Peyronie's disease, is the fibrosis of the cavernous sheaths which causes the retraction of the covering band of the cavernous bodies, as well as leading to a deviated and painful erection. The aetiology of this disease is unknown. The retraction usually causes the deviation of the erect penis towards the side which is affected by the fibrosis. This pathology causes patients to have painful erections and frequently prevents penetration. The results of the treatment are not predictable. The surgical ablation of the fibrotic plaque and its replacement with an implant can be successful, just as it can cause further harm and a worsening of the problem. Local injections of verapamil and corticosteroids with high anti-inflammatory power can be effective, but taking the corticosteroids orally, which, as already known, has a series of side effects and contraindications, is not effective.

There is thus the necessity of being able to have a therapy which is effective for such a type of dysfunctions, which therefore does not have the drawbacks of the therapies known to this day of the aforementioned sexual dysfunctions.

WO 2010/058576 describes adhesive compositions to be applied onto a support made from fabrics for bandages containing zeolites, in which NO is incorporated.

Zeolites are indeed particular aluminosilicates in which alumina and silica are chemically bound to one another so as to provide a rigid crystalline mesh containing empty spaces that are capable of incorporating a gas, such as in particular NO and subsequently of releasing it if necessary.

NO is indeed a gas which plays a main role in many physiological mechanisms amongst which also the erection.

It is also known to use fibres containing particular mixtures of powders which may or may not be metallic, capable of emitting radiation with beneficial properties.

For example US 5,258,558 describes a powder capable of emitting radiation in the infrared and fibres containing said powder. The powder is mainly made up of alumina, titanium metal or platinum silica or palladium metal. These fibres, suitably processed in yarns, are used for preparing fabrics with thermoregulating characteristics which are beneficial to the human body and can therefore be used both for the preparation of fabrics for clothing, and for linen such as sheets, covers etc.

In particular this powder is 30-45% made up of alumina with a granulometry < 1 m and 70-55% by weight is made up of titanium with a granulometry thereof in the order of magnitude of that of alumina and finally platinum or palladium in the colloidal form therefore with a granulometry of between 7 and 40Å.

Unexamined Japanese patent applications, JP04073226, JP03-241025 also describe preparation processes of such fibres as well as of end yarns, and in these processes titanium in the metal form is replaced by titanium dioxide.

Patent application EP 1291405 describes fibres that are similar to those described in the previous patents with the single substantial difference that the base powdered composition also contains small amounts of silver so as to give the fibres also anti-static properties. WO 2010/058176 A2 describes use of a dressing composition comprising i) an elastomeric-adhesive composition and ii) a zeolite (aluminosilicate) comprising releasable adsorbed nitric oxide, wherein the composition can be used in the treatment of erectile dysfunction. Some of the fibres containing the compositions capable of emitting radiation, described in the aforementioned patent document are available on the market with the NEXUS ES^{®} trademark.

### SUMMARY OF THE PRESENT INVENTION

The present invention regards the use as claimed in claim 1.

In fact the applicant has now surprisingly found that fibres containing from 0.005 to 10% of a powdered composition (A) that is capable of emitting radiation in the far infrared, as the only further component, said powdered composition (A) consisting of a mixture of:
a) alumina, in concentrations of between 25 and 55% by weight on the total weight of the powdered composition
b) titanium dioxide in a quantity of between 75 and 45% by weight on the total weight of the composition
c) at least one metal or a compound thereof selected among: Pt in a quantity of between 0.001 and 0.1% by weight on the total weight of the composition,
once transformed into yarns and woven so as to prepare male underwear, or processed with the purpose of obtaining non-woven fabric, are particularly effective in the topical treatment of sexual dysfunction of the male genital apparatus selected from the erectile dysfunction or deficit (ED) and the Peyronie's disease or Induratio Penis Plastica(IPP).

### DESCRIPTION OF THE FIGURES

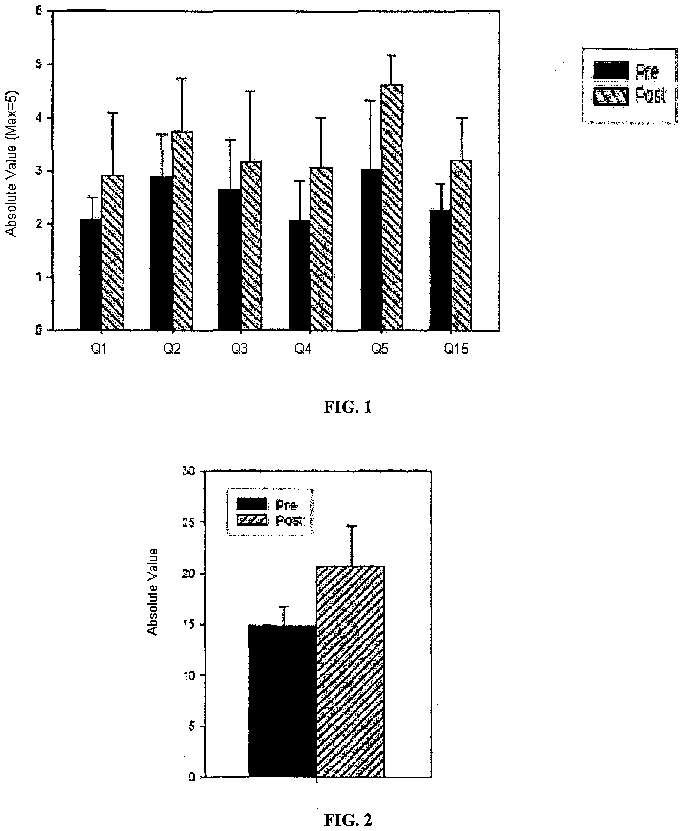

Figure 1 represents the partial variation of the erection scores (IEFF) on patients affected by ED, following the treatment with clothing containing the fibres for use according to the present invention, where the ordinate gives the absolute numerical value, and the abscissa gives the number of the questions relative to the specific pathology (Q1-5,15), which, the patients undergoing the treatment with underwear according to the invention, answered.
Figure 2 on the other hand shows in the form of a graph the sum of the IIEFF score results, of the data of the graph in figure 1.

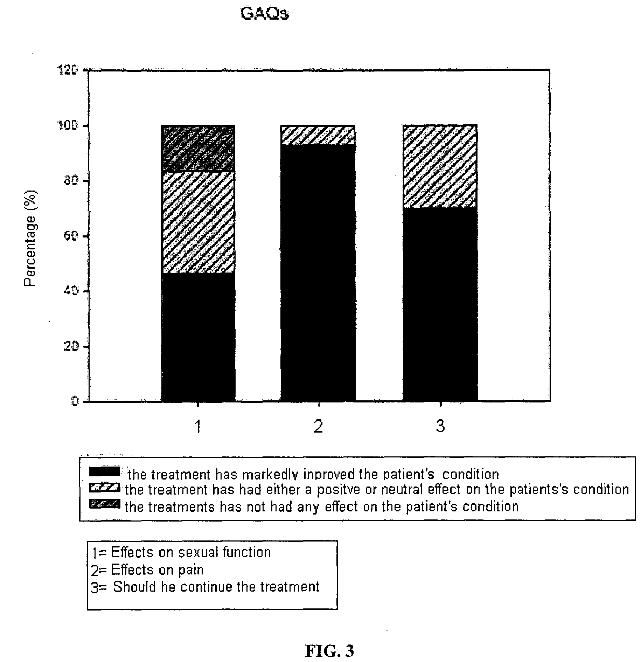

Figure 3 shows the general self-evaluation values following treatment with clothing containing the fibres according to the present invention, where the ordinate gives the percentages whereas the abscissa gives the questions asked to the patients concerning the effectiveness of the treatment about effects on sexual function (only to the patients affected by ED), effects on pain (only to patients affected by IPP), and if the patient would or would not continue the treatment (to all patients without distinction).

### DETAILED DESCRIPTION OF THE INVENTION

The fibres for the use object of the present invention can be artificial fibres and therefore, in addition to the composition capable of emitting radiation in the far infrared, they contain polymers of the conventional type and, preferably, at least one selected among polyester, polyurethane such as for example the product generally known with the Elastam ® trademark, polyamides such as for example Nylon®; but can also be natural fibres and therefore, in addition to the aforementioned composition they can also consist of, for example, cotton fibres, flax, hemp. According to one particularly preferred solution the fibres for use according to the present invention are artificial fibres which in addition to containing the compositions capable of emitting radiation in the far infrared, are made up of polyester.

For the purposes of the present invention, by fibre or fibre tuft, both of natural or artificial origin, we mean that intermediate product, which, before being used for textile uses, must undergo spinning processes, for which one or more fibres are transformed into yarn.

The aforementioned fibres, both of the natural and artificial type, can also be transformed into a non-woven fabric, therefore they are joined to one another and are transformed into a fabric, not with weaving processes but with thermal processes or gluing, or punching with needles, laser, etc.

The fibres for use according to the present invention comprise the compositions capable of emitting radiation in the far IR in concentrations of between 0.005 and 10% by weight on the total weight of the fibres.

The powdered compositions contained in the fibres for the use according to the present invention contain the component (a), alumina, in amounts of between 25 and 55% by weight on the total weight of the powdered composition.

The component (b), titanium dioxide, is, on the other hand, present in amounts that are comprised between 75 and 45% by weight on the total weight of the composition. Finally, the component (c) platinum metal, is comprised between 0.001 and 1% by weight on the total weight of the composition.

The compositions can possibly also contain other transition metals or relative compounds, such as for example silver or copper with the purpose of reducing the electrostatic charges of the fibres. The granulometric dimensions of the components (a) and (b) and (c) of the powder contained in the fibres for use according to the present invention are those described in patent US 5,258,558.

According to a preferred embodiment of the present invention the fibres used are those available on the market with the NEXUS ES^{®} trademark.

The yarns obtained with the fibres according to the present invention can in turn be woven with yarns of the conventional type such as, for example, yarns in natural cellulose material preferably selected from at least one of the following types: cotton, flax, hemp, and/or with artificial yarns preferably selected from at least one of the following types polyester, polyamide, polyurethane.

The fabrics thus obtained are used for manufacturing male underwear and, for the purposes of the present invention, are covered by such a category for example underpants, briefs, boxer shorts, leotards, close-fitting underwear, half leg underwear, etcetera.

The non-woven fabric, in addition to the fibres for use according to the present invention, can also contain conventional artificial fibres containing polymers preferably selected from at least one of the following types: polyester, polyamide and polyurethane, and/or natural fibres preferably at least one selected from cotton, hemp, flax.

A further object of the present invention is also an insert containing non-woven fabric comprising artificial and/or natural fibres comprising from 0.005 to 10% by weight on the total weight of the fibre of a powdered composition (A) capable of emitting radiation in the far infrared as the only further component consisting of a mixture of:
a) alumina in concentrations of between 25 and 55% by weight on the total weight of the powdered composition
b) titanium dioxide, in a quantity of between 75 and 45% by weight on the total weight of the composition
c) at least one metal selected among: Pt, in a quantity of between 0.001 and 0.1% by weight on the total weight of the composition and a surface of said non-woven fabric is in contact with one or more external parts of the human body, said insert being provided with at least one element for fastening to an item of clothing of the permanent type, such as for example seams, or temporary type, such as for example automatic buttons and/or Velcro.

The insert object of the present invention can be applied inside of underwear and is made for the treatment of male sexual dysfunctions selected from the aforementioned ED and IPP.

In the rest of the description we shall show with illustrative purposes the clinical tests confirming the therapeutic effectiveness of the fibres for the use according to the present invention.

### 1)Introduction

Vasodilation and myo-relaxation are at the bottom of neuromotor phenomena that are responsible for the erection. It was then desired, with these clinical tests, to verify whether the application of such fibres at the penis level can ensure a better penile oxygenation fighting hypoxia phenomena at the bottom of structural and dysfunctional modifications of the penis which occur with age and when there is comorbidity.

Analogously we also wanted to verify whether such fibres can ensure better oxygenation, promoting the elimination of fibrotic factors, thus making them useful even in the treatment of fibro connective diseases of the penis like, for example, Induratio Penis Plastica, a pathology that is spreading rapidly with an unknown aetiology and of which there is no effective treatment.

### 2) Primary end points of the testing

### (A) Evaluation of the effectiveness of the application of fabric containing the fibres for use according to the present invention on patients affected by ED (12 hours/day for 45 consecutive days on the erectile function measured through IIEF-5 (International Index of Erectile Function

(A-1) Evaluation of the effectiveness of application of the fabric containing the fibres according to the present invention, through the evaluation (Global Assessment Question) as far as sexual function is concerned.

### (B) Evaluation of the application efficiency of the fabric containing the fibres according to the present invention, through evaluation GAQs (Global Assessment Question) on the pain in patients affected by IPP

### Patients- Branch (A)/(affected by ED)

55 patients were preselected and 35 patients affected from ED were recruited in the treatment step.

35 males aged between 22 and 65 years old (average age 42) were provided with supportive underwear (close-fitting briefs or boxer shorts) made from NEXUS ES ^{®} fibres for use according to the present invention. During such a period the patient were not allowed to take any pro-erectile, vasodilator or anti-inflammatory drug. Before the first application (time 0, visit 1) and after 45 days exposed to the fibres for use according to the invention (time 2, visit 2), self-evaluation questionnaires (IEFF and GAQs) were given to the patients concerning the severity of the erectile function.

### Patients branch (B)(affected by IPP)

35 patients were preselected and 18 patients affected by IPP were recruited in the treatment step.

The 18 males aged between 45 and 72 years old (average age 63) were provided with supportive underwear (close-fitting briefs or boxer pants containing NEXUS ES ^{®}) fibres made with the fibres for the use according to the present invention. During such a period the patients were not allowed to use any pro-erectile, vasodilator or anti-inflammatory drugs. Before the first application (time 0, visit 1) and after 45 days of being exposed to the fibres for the use according to the invention (time 2, visit 2), the patients were provided with self-evaluation questionnaires (GAQs).

### 3) Results

### 1. Modification of the IEFF on patients affected by ED following treatment with the underwear garments containing the fibres according to the present invention

The following table shows the numeric values of the IEFF concerning the single questions (Q1-Q5 and Q15), specific of the ED pathology.

| Questionnaire | Pretreatment | StDev | Posttreatment | StDev |
|---|---|---|---|---|
| Q1 | 2.08 | 0.4 | 2.89 | 1.18 |
| Q2 | 2.87 | 0.8 | 3.74 | 1.01 |
| Q3 | 2.63 | 0.9 | 3.18 | 1.31 |
| Q4 | 2.05 | 0.7 | 3.05 | 0.96 |
| Q5 | 3.03 | 1.3 | 4.63 | 0.54 |
| Q15 | 2.26 | 0.5 | 3.21 | 0.78 |
| TOTAL | 14.92 | 1.8 | 20.71 | 3.86 |

Figures 1 and 2 show in a graphical form respectively the results obtained with the treatment according to the present invention for each single question and the sum of such results from which an increase of the erectile function greater than 30% is highlighted. (average of all patients examined).

### A-1) GAQs concerning the effects on sexual function, following treatment with the underwear clothing with the fibres according to the invention.

The following questionnaire was given to the patients to be filled out after the treatment.

*"How would you evaluate the effects of the product used over the last month, based upon your overall sexual function:*
*a) I have experienced an improvement of my sexual function;*
*b) I did not experience any positive or negative effect (neutral effect) ;*
c) *I did not experience any positive effect and*/*or I experienced a worsening and*/*or I would have preferred to use a different product".*

The results of such a test are shown in the first graph of Figure 3

### B) GAQs concerning the effects on the pain carried out on patients affected by IPP, following the treatment with underwear items with NEXUS ES^{®} fibres according to the invention.

How would you judge the effects of the product you have used over the last month, concerning genital pain or discomfort:
*a) I experienced an improvement of the pain symptoms*
*b) I did not experience any positive effect nor any negative effect*
*c) I did not experience any positive effect and*/*or I experienced worsening of the symptoms and*/*or I would have preferred using a different product.*

The results are shown in the second graph of figure 3.

### C) GAQs concerning product preference

The following questionnaire was given to patients to be filled after the treatment:
*"If the product you have used over the last period was immediately available on the market, would you continue to use it:*
   *a) Yes*
   *b) No"*

The results of the aforementioned self-evaluation are shown in a third graph of figure 3.

### D) Analysis of the results

As far as the erectile function is concerned, as can be seen from the GAQS the patients affected by ED evaluated the treatment with the fibres according to the present invention positively in 83% of cases and very positively in 46% of cases). The positive effects moreover documented also with IEFF-5 are in any case greater than 30% of the erectile function score (average of all patients examined). An even more interesting data is that which was obtained for pain (penile, but also scrotal) in IPP patients, with an improvement in 93% of cases.

Overall, as can be seen in the third graph of figure 3, 70% of the individuals would continue the treatment, if it were available.

Moreover, from studies relating to a temporary evaluation of the treatment it has been found that the effectiveness of the fibres according to the present invention has proved to be rapid over some hours in some cases and a few days in most cases, and lasting over time even after finishing the treatment.

## Claims

1. Fibres containing from 0.005 to 10% by weight on the total weight of the fibre of a powdered composition (A) capable of emitting radiation in the far infrared, as the only further component, said powdered composition (A) consisting of a mixture
a) alumina in concentrations of between 25 and 55% by weight on the total weight of the powdered composition
b) titanium dioxide in a quantity of between 75 and 45% by weight on the total weight of the composition
c) at least one metal or a compound thereof selected among: Pt, in a quantity of between 0.001 and 0.1% by weight on the total weight of the composition,
for use in topically treating sexual dysfunction of the male genital apparatus selected from erectile dysfunction (ED) and Induratio Penis Plastica (IPP).

2. Fibres for use according to claim 1, **characterised in that** said fibres are artificial and also contain polymers selected from at least one of the following types: polyesters, polyamides, polyurethanes.

3. Fibres for use according to claim 1 **characterised in that** said fibres are natural and are selected from cotton, flax, hemp.

4. One or more fibres for use according to any one of claims 1-3, wherein they are transformed in yarns by spinning processes.

5. One or more fibres for the use according to claim 4, wherein said yarns are woven with yarns of conventional type selected from natural yarns in natural cellulosic material and/or with artificial yarns thereby obtaining a fabric for manufacturing male underwear.

6. One or more fibres for the use according to claim 5, wherein said conventional type yarns in natural cellulosic material are selected from at least one of the following types cotton, flax and hemp yarns.

7. One or more fibres for the use according to claim 5, wherein said conventional type artificial yarns are selected from at least one of the following types: polyester, polyamide and polyurethane yarns.

8. Fibres for use according to any one of claims 1-3, wherein said fibres are contained in a nonwoven fabric.

9. Fibres for use according to claim 8, wherein said nonwoven fabric contains also conventional artificial fibres and/or natural fibres.

10. Fibres for the use according to claim 8, wherein said non-woven fabric is contained in an insert, with a surface of said non-woven fabric being in contact with one or more external parts of the human body, said insert being provided with at least one permanent or temporary element, fastening to an item of clothing, wherein said clothing item is underwear and said insert is applied inside of said underwear.

## Patentansprüche

1. Fasern, die eine Menge zwischen 0,005 und 10% im Gewicht bezogen auf das Gesamtgewicht der Faser einer pulverförmigen Zusammensetzung (A) enthalten, die in der Lage ist, Strahlung im fernen Infrarot als einzige weitere Komponente zu emittieren, wobei die genannte pulverförmige Zusammensetzung (A) aus einer Mischung aus:
a) Aluminiumoxid in Konzentrationen zwischen 25 und 55% im Gewicht bezogen auf das Gesamtgewicht der pulverförmigen Zusammensetzung,
b) Titandioxid in einer Menge zwischen 75 und 45% im Gewicht bezogen auf das Gesamtgewicht der Zusammensetzung,
c) mindestens einem Metall oder einer Verbindung davon, ausgewählt aus: Pt, in einer Menge zwischen 0,001 und 0,1% im Gewicht bezogen auf das Gesamtgewicht der Zusammensetzung,
zur Verwendung bei der topischen Behandlung der sexuellen Dysfunktionen des männlichen Genitalapparates, ausgewählt aus der erektilen Dysfunktion (ED) und der Induratio Penis Plastica (IPP), besteht.

2. Fasern zur Verwendung nach Anspruch 1, **dadurch gekennzeichnet, dass** die Fasern künstlich sind und auch Polymere enthalten, die aus mindestens einem der folgenden Typen: Polyester, Polyamide, Polyurethane ausgewählt werden.

3. Fasern zur Verwendung nach Anspruch 1, **dadurch gekennzeichnet, dass** die Fasern natürlich sind und aus Baumwolle, Flachs, Hanf ausgewählt werden.

4. Eine oder mehrere Fasern zur Verwendung nach einem der Ansprüche 1 bis 3, wobei sie durch Spinnverfahren in Garnen umgewandelt werden.

5. Eine oder mehrere Fasern zur Verwendung nach Anspruch 4, wobei die genannten Garne mit aus natürlichen Garnen aus natürlichem Zellulosematerial ausgewählten herkömmlichen Garnen und/oder mit künstlichen Garnen gewebt werden, wodurch ein Gewebe zur Herstellung von männlicher Unterwäsche erhalten wird.

6. Eine oder mehrere Fasern zur Verwendung nach Anspruch 5, wobei die genannten herkömmlichen Garne aus natürlichem Zellulosematerial aus mindestens einem der folgenden Typen: Baumwoll-, Flachs- und Hanfgarne ausgewählt werden.

7. Eine oder mehrere Fasern zur Verwendung nach Anspruch 5, wobei die genannten herkömmlichen künstlichen Garne aus mindestens einem der folgenden Typen: Polyester-, Polyamid- und Polyurethangarne ausgewählt werden.

8. Fasern zur Verwendung nach einem der Ansprüche 1-3, wobei die Fasern in einem Vliesstoff enthalten sind.

9. Fasern zur Verwendung nach Anspruch 8, wobei der genannte Vliesstoff auch herkömmliche künstliche Fasern und/oder natürliche Fasern enthält.

10. Fasern zur Verwendung nach Anspruch 8, wobei der genannte Vliesstoff in einem Einsatz enthalten ist, indem eine Oberfläche des Vliesstoffs in Kontakt mit einem oder mehreren äußeren Teilen des menschlichen Körpers steht, wobei der Einsatz mit mindestens einem permanenten oder temporären Element versehen ist, das an einem Kleidungsstück befestigt ist, wobei das genannte Kleidungsstück Unterwäsche ist und der genannte Einsatz innerhalb der Unterwäsche angebracht wird.

## Revendications

1. Fibres contenant de 0,005 à 10% en poids sur le poids total de la fibre d'une composition en poudre (A) capable d'émettre des rayonnements dans l'infrarouge lointain en tant que seul autre composant, ladite composition en poudre (A) consistant en un mélange de
a) alumine à des concentrations comprises entre 25 et 55% en poids sur le poids total de la composition en poudre,
b) dioxyde de titane en une quantité comprise entre 75 et 45% en poids sur le poids total de la composition,
c) au moins un métal ou l'un de ses composés choisi parmi : Pt, en une quantité comprise entre 0,001 et 0,1 % en poids sur le poids total de la composition,
pour une utilisation dans le traitement topique des dysfonctions sexuelles de l'appareil génital masculin choisi entre la dysfonction érectile (ED) et l'Induratio Penis Plastica (IPP).

2. Fibres à utiliser selon la revendication 1, **caractérisées en ce que** lesdites fibres sont artificielles et contiennent également des polymères choisis parmi au moins un des types suivants : les polyesters, les polyamides, les polyuréthanes.

3. Fibres à utiliser selon la revendication 1, **caractérisées en ce que** lesdites fibres sont naturelles et sont choisies parmi le coton, le lin, le chanvre.

4. Une ou plusieurs fibres à utiliser selon l'une quelconque des revendications 1 à 3, où elles sont transformées en fil par des procédés de filage.

5. Une ou plusieurs fibres pour l'utilisation selon la revendication 4, où lesdits fils sont tissés avec des fils de type classique choisis parmi des fils naturels en matière cellulosique naturelle et/ou avec des fils artificiels, obtenant ainsi un tissu pour la fabrication de sous-vêtements masculins.

6. Une ou plusieurs fibres pour l'utilisation selon la revendication 5, où lesdits fils de type classique en matière cellulosique naturelle sont choisis parmi au moins un des types suivants : fils de coton, de lin et de chanvre.

7. Une ou plusieurs fibres pour l'utilisation selon la revendication 5, où lesdits fils artificiels de type classique sont choisis parmi au moins un des types suivants : fils de polyester, de polyamide et de polyuréthane.

8. Fibres à utiliser selon l'une quelconque des revendications 1 à 3, où lesdites fibres sont contenues dans un tissu non-tissé.

9. Fibres à utiliser selon la revendication 8, où ledit tissu non-tissé contient également des fibres artificielles classiques et/ou des fibres naturelles.

10. Fibres pour l'utilisation selon la revendication 8, où ledit tissu non-tissé est contenu dans un insert, une surface dudit tissu non tissé étant en contact avec une ou plusieurs parties externes du corps humain, ledit insert étant pourvu d'au moins un élément permanent ou temporaire, fixé à un article de vêtement, où ledit article de vêtement est un sous-vêtement et ledit insert est appliqué à l'intérieur dudit sous-vêtement.
